# EUROPEAN PATENT APPLICATION

(11) **EP 4 647 063 A1**
(43) Date of publication of application: **12.11.2025**
(21) Application number: 24174615.5
(22) Date of filing: 07.05.2024
(51) Int. Cl.: A61K 8/34, A61K 8/49, A61K 8/64, A61K 8/67, A61K 8/68, A61K 8/73, A61Q 19/00, A61Q 19/08

(54) **COSMETIC PROCEDURE FOR THE OPTIMIZATION OF AESTHETIC PARAMETERS OF THE SKIN AND SKIN APPENDAGES**

(71) Applicant: Etichub S.r.l., 27100 Pavia (IT)
(72) Inventor: BLEVE, Mariella, 27028 SAN MARTINO SICCOMARIO (IT); GRIGNANI, Camilla, 27100 PAVIA (IT); PERUGINI, Paola, 27049 STRADELLA (IT)
(74) Representative: Lunati & Mazzoni S.r.L.

(57) **Abstract**

A cosmetic procedure is envisaged for the optimization of aesthetic parameters of the skin and skin appendages carried out over a period of 28 days, wherein the period of 28 days includes a first week corresponding to a menstrual phase, a second week corresponding to a follicular phase, and a third and fourth week corresponding to a luteal phase; wherein the procedure includes, during the first and fourth week, cleansing and treating the skin or skin appendages with respectively a first and a second mixture including at least polyphenolic structures contained in plant extracts, unsaponifiable substances of oils or fats, provitamins, vitamins and/or derivatives of group B; and, during the second and third week, cleansing and treating the skin or skin appendages with respectively a third and a fourth mixture including at least biomimetic structures, natural or natural origin polymers, unsaponifiable substances of oils or fats, provitamins, vitamins and/or derivatives of groups C and E, and wherein the second and fourth mixture further include Ectoine and Sodium Hyaluronate.

## Description

The present invention relates to a cosmetic procedure for the optimization of aesthetic parameters of the skin and skin appendages of the type specified in the preamble of the first claim. In particular, this invention relates to a cosmetic procedure suitable for use in contexts that, in addition to facial skin, encompass the body, the intimate area, the scalp, skin appendages, and generally every area wherein application of cosmetic products occurs.

As known, understanding the profound influence that fluctuating hormone levels have on biological responses in women is crucial for optimizing the efficacy of cosmetological choices because, although the changes may appear mild and imperceptible, skin actually changes over a span of twenty-eight days. These hormonal changes, also responsible for the menstrual cycle, have a significant action on the female body with known impacts both physically and emotionally.

In particular, literature review shows that menstruation is governed by changes orchestrated by the levels of female hormones (estrogens and progesterone) with different reactions in various tissues and organs including the skin. The latter is rich in estrogen receptors, both at the dermal and epidermal level, and to a lesser extent, in progesterone receptors expressed, together with those of androgens, in keratinocytes, fibroblasts, and macrophages. Cyclically fluctuating levels of estrogens and progesterone influence numerous characteristics of the epidermis, including the lipid secretion of the skin surface, sebum production, skin thickness, skin hydration, and barrier function. The collagen content, contributing to skin elasticity and resistance to wrinkle formation, is also influenced. Estrogens are associated with collagen synthesis, increase thickness and water content through the production of hyaluronic acid, improve barrier function and promote the restoration of barrier integrity after a damage. It is also interesting to note that estrogen levels affect the microflora as well as the pigmentation of the skin and susceptibility to UV rays; estrogens stimulate melanogenesis; indeed, premenstrual hyper-pigmentation can often occur.

A month can be divided into different phases of the menstrual cycle depending on the levels of the cycle protagonists, secreted day by day. The distinct phases encountered over the course of a single month are: the menstrual phase, the follicular phase, and the luteal phase.

During the menstrual phase, the menstrual cycle begins with the onset of menstruation and continues for about four or six days. During this phase, defined as "the first week", the body produces more hormone prostaglandin which increases pain sensitivity, while estrogen levels are quite low.

The follicular phase, starting from the seventh to the fourteenth day, culminates with ovulation. It is a distinct and more important phase from a dermatological standpoint because the body restarts the production of estrogens, a process that enriches the new skin cells that are forming at this time. The main source of estrogens is the ovary which produces several types of estrogens, among which estradiol is the most powerful. The production of estradiol steadily increases in the week preceding ovulation and peaks one day before ovulation. At the ovulatory moment, the production of estrogens and luteinizing hormone reaches its peak.

In the luteal phase, ovulation ends with the formation of the corpus luteum which produces progesterone in the week following ovulation. Estradiol increases again in the luteal phase and has a second peak, five days after ovulation, then lowers while preparing for menstruation. In the final stages of the luteal phase, the levels of estrogen and progesterone decrease, giving rise to menstruation and providing a new start to the cycle. The pre-menstrual syndrome is fully started.

At present, both home and professional cosmetic treatments do not allow women to follow a cosmetic routine that puts them in ideal conditions of well-being for skin and/or skin appendages. In fact, there are no cosmetics on the market capable of responding to the aforementioned hormonal needs. Furthermore, there is a clear lack of specificity of use on the products to which one might refer; stand-alone cosmetics, ideally belonging to cosmetic lines born for different purposes, can only partially respond, more or less inadequately, to the needs of the skin and/or skin appendages over the hormonal month. The validity of this principle is further accentuated when the task of understanding one's own skin needs is delegated to the consumer and, consequently, of selecting the sequence of products most suitable for achieving the desired care goals. Therefore, the formulative proposals and related procedures on the market are lacking in guaranteeing correct and effective product use, not fully satisfying the performance needs and sensory care experience of women.

In this situation, the technical task at the base of the present invention is to devise a cosmetic procedure for the optimization of aesthetic parameters of the skin and skin appendages that can substantially obviate at least part of the cited inconveniences.

Within this technical task, an important purpose of the invention is to obtain a cosmetic procedure for the optimization of aesthetic parameters of the skin and skin appendages that is capable of selectively counteracting the alterations of the skin and skin appendages caused, for example, by changes related to the menstrual cycle and/or by hormonal fluctuations in order to restore the ideal conditions of the skin and/or skin appendages.

Another important purpose of the invention is to implement a cosmetic procedure for the optimization of aesthetic parameters of the skin and skin appendages that allows to relieve the consumer from such a responsibility, in particular by sparing him the task of understanding its own skin needs.

The technical task and the specified aims are achieved by a cosmetic procedure for the optimization of aesthetic parameters of the skin and skin appendages as claimed in the attached claim 1.

Preferred technical methods are highlighted in the dependent claims.

The features and advantages of the invention are further clarified by the detailed description of preferred embodiments of the invention, with reference to the attached drawings, wherein:

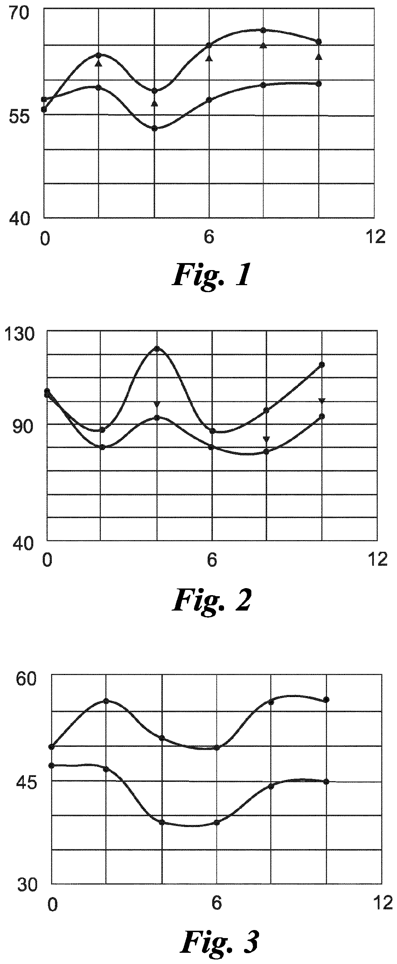

**Fig. 1** shows a graph related to the hydration of the skin obtained by implementing the cosmetic procedure for the optimization of aesthetic parameters of the skin and skin appendages according to the invention on a sample of eight women over ten weeks; in such a graph, the number of weeks is present on the abscissa, on the ordinate the hydration in arbitrary units obtained, the upper curve describes the hydration obtained with the procedure, and the lower curve the hydration obtained with conventional methods with a placebo;
**Fig. 2** illustrates a graph related to the production of skin sebum obtained by implementing the cosmetic procedure for the optimization of aesthetic parameters of the skin and skin appendages according to the invention on a sample of eight women over ten weeks; in this second graph, the number of weeks is present on the abscissa, the amount of sebum in µg/cm2 is present on the ordinate, while the upper curve describes the sebum obtained with conventional methods with a placebo and the lower curve the sebum obtained with the procedure;
**Fig. 3** is a graph related to the count of skin impurities obtained by implementing the cosmetic procedure for the optimization of aesthetic parameters of the skin and skin appendages according to the invention on a sample of eight women over ten weeks; in this third graph, the number of weeks is present on the abscissa, the quantity of impurities on the ordinate, the upper curve describes the number of impurities obtained with conventional methods with a placebo, and the lower curve the number of impurities obtained with the procedure.

In this document, measures, values, shapes, and geometric references (such as perpendicularity and parallelism), when associated with words like "approximately" or other similar terms such as "almost" or "substantially", are to be understood as allowing measurement errors or inaccuracies due to production and/or manufacturing errors, and above all, a slight deviation from the value, measure, shape, or geometric reference to which it is associated. For example, such terms, when associated with a value, preferably indicate a deviation not exceeding 10% of the value itself.

Moreover, when used, terms such as "first", "second", "upper", "lower", "main", and "secondary" do not necessarily identify an order, a priority of relationship, or relative position, but may simply be used to more clearly distinguish different components from one another.

Measurements and data reported in this text are to be considered, unless otherwise indicated, as obtained in International Standard Atmosphere ICAO (ISO 2533:1975).

The cosmetic procedure for the optimization of aesthetic parameters of the skin and skin appendages is aimed at normalizing and enhancing the appearance of the skin of the face and/or body, scalp, hair, mucous membranes, nails, and collectively, every area of application of cosmetic products.

The procedure is carried out over a period of 28 days.

The period of 28 days substantially corresponds to the menstrual cycle for a female individual.

Thus, the period substantially includes at least a first week, a second week, a third week, and a fourth week. The first week preferably corresponds to a menstrual phase; the second week preferably corresponds to a follicular phase; the third and fourth week preferably correspond to a luteal phase.

The procedure also includes cleansing and treatment phases. Advantageously, during the first and fourth week, the cleansing of the skin or skin appendages is carried out with a first mixture.

The first mixture includes at least polyphenolic structures contained in plant extracts, unsaponifiable substances of oils or fats, and provitamins, vitamins and/or derivatives of group B.

The first mixture might also include biomimetic structures and/or natural or natural origin polymers and/or provitamins, vitamins and/or derivatives of groups C and E.

The biomimetic structures, as just described and subsequently detailed, can include one or more among glycolipids, phospholipids, amino acids, and peptides.

In any case, preferably, polyphenolic structures contained in plant extracts, unsaponifiable substances of oils or fats, and provitamins, vitamins and/or derivatives of group B are predominant in the first mixture.

Even more in detail, the polyphenolic structures can be included in the first mixture in quantities ranging from 0.02% to 2% by weight of the first mixture. The unsaponifiable substances can be included in the first mixture in quantities ranging from 0.05% to 2% by weight of the first mixture.

Thus, the provitamins, vitamins and/or derivatives of group B can be included in the first mixture in quantities ranging from 0.05% to 10% by weight of the first mixture.

Advantageously, during the first and fourth week, the treatment of the skin or skin appendages is carried out also with a second mixture. In particular, during the first and fourth week, the treatment of the skin or skin appendages is carried out respectively with a first and a second mixture.

Similarly to the first mixture, the second mixture includes at least polyphenolic structures contained in plant extracts, unsaponifiable substances of oils or fats, and provitamins, vitamins and/or derivatives of group B.

The second mixture might also include biomimetic structures and/or natural or natural origin polymers and/or provitamins, vitamins and/or derivatives of groups C and E.

In any case, preferably, polyphenolic structures contained in plant extracts, unsaponifiable substances of oils or fats, and provitamins, vitamins and/or derivatives of group B are predominant in the second mixture.

Even more in detail, the polyphenolic structures can be included in the second mixture in quantities ranging from 0.5% to 15% by weight of the second mixture. The unsaponifiable substances can be included in the second mixture in quantities ranging from 0.2% to 10% by weight of the second mixture.

Thus, the provitamins, vitamins and/or derivatives of group B can be included in the second mixture in quantities ranging from 0.1% to 5% by weight of the second mixture.

Unlike the first mixture, the second mixture further includes Ectoine and Sodium Hyaluronate.

In detail, Ectoine can be included in the second mixture in quantities ranging from 0.2% to 5% by weight of the second mixture.

Thus, Sodium Hyaluronate can be included in the second mixture in quantities ranging from 0.02% to 10% by weight of the second mixture.

Advantageously, during the second and third week, the cleansing of the skin or skin appendages is carried out with a third mixture.

The third mixture includes at least biomimetic structures, natural or natural origin polymers, unsaponifiable substances of oils or fats, and provitamins, vitamins and/or derivatives of groups C and E.

The third mixture might also include polyphenolic structures contained in plant extracts and/or provitamins, vitamins and/or derivatives of group B.

In any case, preferably, biomimetic structures, natural or natural origin polymers, unsaponifiable substances of oils or fats, and provitamins, vitamins and/or derivatives of groups C and E are predominant in the third mixture.

Even more in detail, the biomimetic structures can be included in the third mixture in quantities ranging from 0.5% to 30% by weight of the third mixture.

The polymers can be included in the third mixture in quantities ranging from 0.5% to 20% by weight of the third mixture.

The unsaponifiable substances can be included in the third mixture in quantities ranging from 0.5% to 10% by weight of the third mixture.

Thus, the provitamins, vitamins and/or derivatives of groups C and E can be included in the third mixture in quantities ranging from 0.02% to 10% by weight of the third mixture.

Advantageously, during the second and third week, the treatment of the skin or skin appendages is carried out using a fourth mixture.

Similarly to the third mixture, the fourth mixture includes at least biomimetic structures, natural or natural origin polymers, unsaponifiable substances of oils or fats, and provitamins, vitamins and/or derivatives of groups C and E.

The fourth mixture might also include polyphenolic structures contained in plant extracts and/or provitamins, vitamins and/or derivatives of group B.

In any case, preferably, biomimetic structures, natural or natural origin polymers, unsaponifiable substances of oils or fats, and provitamins, vitamins and/or derivatives of groups C and E are predominant in the fourth mixture.

Even more in detail, the biomimetic structures can be included in the fourth mixture in quantities ranging from 2% to 30% by weight of the fourth mixture. The polymers can be included in the fourth mixture in quantities ranging from 0.02% to 15% by weight of the fourth mixture.

The unsaponifiable substances can be included in the fourth mixture in quantities ranging from 0.5% to 30% by weight of the fourth mixture.

Thus, the provitamins, vitamins and/or derivatives of groups C and E can be included in the fourth mixture in quantities ranging from 0.5% to 25% by weight of the fourth mixture.

Unlike the third mixture, the fourth mixture further includes Ectoine and Sodium Hyaluronate.

In detail, Ectoine can be included in the fourth mixture in quantities ranging from 0.01 % to 5% by weight of the fourth mixture.

Thus, Sodium Hyaluronate can be included in the fourth mixture in quantities ranging from 0.1% to 5% by weight of the fourth mixture.

In a preferred, but not exclusive, embodiment, the first mixture preferably comprises flowers/leaves/stems water of at least one among and preferably the entirety of Angelica and Melissa.

Thus, the second mixture preferably includes at least one among and more preferably the entirety of Melissa officinalis flower/leaf/stem water, Vitis vinifera seed oil, Helianthus annuus seed oil, punica granatum extract, oleanolic acid, jojoba esters, Angelica archangelica extract, Melissa officinalis extract, Euphorbia cerifera wax, Plantago lanceolata extract, Mahonia aquifolium extract, Enantia chlorantha extract, xanthan gum, yogurt extract, sodium salicylate, Ectoine, and Sodium Hyaluronate.

The third mixture preferably includes at least one among and more preferably the entirety of Olea europaea oil, Vitis vinifera seed oil, glycolipids, Helianthus annuus seed oil, Curcuma longa extract, sodium PCA, tocopherol, xanthan gum, potassium olivoyl PCA.

The fourth mixture preferably comprises flowers/leaves/stems water of at least one among and more preferably the entirety of Melissa officinalis, Helianthus annuus seed oil, Pistacia vera seed oil, Ribes nigrum extract, jojoba esters, xanthan gum, Euphorbia cerifera wax, Ectoine, and Sodium Hyaluronate.

This invention also introduces a cosmetic kit for the optimization of aesthetic parameters of the skin and skin appendages aimed at normalizing and enhancing the appearance of the skin of the face and/or body, scalp, hair, mucous membranes, nails, and collectively, every area of application of cosmetic products.

Said kit is therefore configured to be used during the above-described procedure in accordance with the above disclosure.

Said kit includes said first mixture, said second mixture, said third mixture, and said fourth mixture. For the composition of each of the mixtures, reference is made to what has been described above regarding each of them.

To demonstrate the beneficial effects of the procedure and thus of the cosmetic kit according to the invention, an instrumental study of characterization of skin and/or skin appendages was preliminarily conducted involving an applicative research phase on a panel of two hundred healthy volunteers, subject to a controlled diet, who were sampled for skin analysis and thus divided into groups in relation to the phase of the menstrual cycle to which they belonged (fertile women - 1st, 2nd, 3rd, and 4th week of cycle; menopause). The instrumental evaluation of the characteristics of the skin and/or skin appendages of the panel in question allowed to identify a scientific rationale on which to base the innovative cosmetic procedure and, thus, the kit according to the invention. Skin hydration is also closely related to the presence of estrogens in the blood. In particular, estradiol is known to increase the levels of mucopolysaccharides and hyaluronic acid in the dermis, molecules that, thanks to their particular structure, retain a large amount of reserve water.

In order to evaluate the variations in skin hydration during the different moments of the menstrual cycle, an instrumental evaluation was performed using the Corneometer CM 825 (Cutometer MPA580, Courage & Khazaka, Cologne, Germany).

Corneometry is a technology used to assess the hydration of the outermost layer of the epidermis: the stratum corneum. Since the skin is a dielectric medium, changes in skin capacitance allow to measure all hydration variations. The instrument used is equipped with a probe having a surface at about 49 mm2, which allows for precise measurements within a second at a depth of 10-20 µm in the stratum corneum. The parameter is expressed according to an arbitrary scale (0-100 U.A.).

The instrumental evaluation is performed at controlled temperature and humidity (T 22°C, R.H. = 50% ± 5%) where volunteers are acclimated for at least 15 minutes. The chosen acquisition area is the cheekbone. 60 subjects aged between 32 and 67 years were recruited as follows:
- FERTILE WOMEN:
   N.10 subjects declare to be in the first week of the cycle, menstrual phase;
   N.10 subjects declare to be in the second week of the cycle, follicular phase;
   N.10 subjects declare to be in the third week of the cycle, luteal phase;
   N.10 subjects declare to be in the fourth week of the cycle, luteal phase.
- WOMEN IN MENOPAUSE:
N. 20 subjects declare to be in menopause.

The following table (Tab.1) reports the average hydration values obtained.

| Table 1: Average hydration values (U.A) of all subjects | | | |
|---|---|---|---|
| Group | Menstrual cycle phase | Age of subjects | Hydration (U.A.) ± Std.Dev. |
| FERTILE WOMEN | 1st week | 37-53 | 45.54 ± 3.82 |
| | 2nd week | 39-46 | 61.67 ± 5.86 |
| | 3rd week | 32-50 | 54.34 ± 3.54 |
| | 4th week | 43-48 | 53.85 ± 11.47 |
| WOMEN IN MENOPAUSE | Absence | 51-67 | 43.83 ± 4.16 |

The results obtained by the use of the Corneometer (Courage & Khazaka) have shown that in fertile women, for example, the skin is well hydrated (61.67 u.a.) in the follicular phase (2nd week) characterized by the maximum concentration of estrogens caused by ovulation.

In the following weeks, 3rd and 4th week, estrogen levels are quite low so that the skin is drier. In particular, hydration undergoes a progressive reduction (54.35 u.a. and 53.85 u.a.) and reaches the lowest value (45.54 u.a.) in the menstrual phase. In this case, a reduction by 26% of the skin hydration parameter compared to the follicular phase is observed.

Water content values of the stratum corneum found in the first week of the cycle are comparable to those obtained in menopausal women where the absence of ovarian activity is responsible for the poor presence of the main female hormones.

The fluctuating levels of female hormones during the menstrual cycle are responsible for the superficial appearance of the skin in the different weeks of the month and in menopause. In detail, estrogens promote the synthesis of collagen, hyaluronic acid, and glycosaminoglycans, encourage skin hydration, and improve the skin barrier function. For these reasons, in the presence of high levels of estrogens, the skin texture appears smoother and smoother, whereas low percentages of the same hormones are responsible for a more irregular skin texture. The evaluation of skin texture was carried out using the PRIMOSpico apparatus (GFM, Teltow, Germany), an optical analysis system that allows to capture a 3D image of the skin surface and to transform it, by appropriate software, in order to measure the topographic parameters of the skin.

In particular, the parameter that characterizes the microrelief considered in this study is the Ra parameter, i.e., the average roughness value, which represents the integral of the function that defines the curve of the skin profile and corresponds to the area that the curve includes above and below its mean line. The higher the Ra value, the greater the skin roughness.

The instrumental evaluation is performed at controlled temperature and humidity (T 22°C, R.H. = 50% ± 5%) whereat volunteers are acclimated for at least 15 minutes. The chosen acquisition area is the cheekbone.

60 subjects aged between 32 and 67 years were recruited as follows:
- FERTILE WOMEN:
   N.10 subjects declare to be in the first week of the cycle, menstrual phase;
   N.10 subjects declare to be in the second week of the cycle, follicular phase;
   N.10 subjects declare to be in the third week of the cycle, luteal phase;
   N.10 subjects declare to be in the fourth week of the cycle, luteal phase.
- WOMEN IN MENOPAUSE:
N. 20 subjects declare to be in menopause.

The following table (Tab.2) reports the hydration data obtained.

| Table 2: Average Ra values (µm) of all subjects | | | |
|---|---|---|---|
| Group | Menstrual cycle phase | Age of subjects | Hydration (U.A.) ± Std.Dev. |
| FERTILE WOMEN | 1st week | 39-53 | 31.3 ± 2.8 |
| | 2nd week | 32-50 | 25.3 ± 2.4 |
| | 3rd week | 43-51 | 28.0 ± 1.8 |
| | 4th week | 43-48 | 26.6 ± 5.1 |
| WOMEN IN MENOPAUSE | Absence | 51-58 | 32.33 ± 3.27 |

From the data obtained, it can be seen how the skin microrelief appears more uniform and smoother in the follicular phase (25.30 µm) compared to the luteal phase (28.00 and 29.60 µm) and the menstrual phase (31.30 µm). The skin texture improves by about 24%.

The values of surface roughness are even higher in menopausal women where constant and scarce levels of estradiol, associated with physiological aging, lead to a progressive depletion of molecules such as collagen, hyaluronic acid, and GAGs responsible for skin turgor.

Skin elasticity is the result of a complex balance strictly dependent not only on estradiol concentrations in the blood but also on various factors, including skin hydration, barrier integrity, and the presence of sebum. Only in menopausal women does skin elasticity seem severely compromised by low levels of estradiol that lead to the alteration of the structure and number of elastin fibers of the skin. In the study, the FERTILE WOMEN group was compared with the MENOPAUSAL WOMEN group without making further subdivisions, as estradiol fluctuations in different weeks of the cycle do not seem so massive as to interfere with the viscoelastic properties of the dermis.

40 subjects aged between 46 and 54 years were recruited as follows:
- FERTILE WOMEN:
   N.20 subjects who are in the three different phases of the menstrual cycle (random recruitment);
- WOMEN IN MENOPAUSE:

   N.20 subjects declare to be in menopause.

The measurement of skin elasticity parameters was carried out through the use of the Cutometer MPA580 (Courage & Khazaka, Cologne, Germany), an instrument that measures the vertical deformation of the skin induced by suction, expressed in mm. A negative pressure of 450 mbar is applied via a probe having a diameter of 2mm on the skin for a time that varies from 1 to 3 seconds. Each suction is followed by a release time that allows the skin to return to the resting condition.

The indicators obtained are:
- compactness (R0): the passive behaviour of the skin after the application of negative pressure;
- return to baseline state (R1): the ability of the skin to return to its original state after the application of negative pressure;
- total elasticity (R2): the resistance against returning to the resting condition at the end of the suction;
- net elasticity (R5): the ratio between the maximum skin extension and the residual skin deformation.

The instrumental evaluation is performed at controlled temperature and humidity (T 22°C, R.H. = 50% ± 5%) where at volunteers are acclimated for at least 15 minutes. The chosen acquisition area is the cheekbone.

The following table (Tab.3) reports the data of R0, R1, R2, R5.

| **Table 3: Average values of R0, R2, R5 for all subjects** | | | | | |
|---|---|---|---|---|---|
| Group | Menstrual cycle phase | R0 ± Std.Dev. | R1 ± Std.Dev. | R2 ± Std.Dev. | R5 ± Std.Dev. |
| FERTILE WOMEN | Random | 0.136 ± 0.032 | 0.023 ± 0.007 | 0.821 ± 0.072 | 0.794 ± 0.163 |
| WOMEN IN MENOPAUSE | Absence | 0.170 ± 0.031 | 0.072 ± 0.016 | 0.573 ± 0.079 | 0.348 ± 0.062 |

During menopause, a rapid decrease in collagen and elastin fibers leads to a progressive increase in skin extensibility (R0), a reduction in elasticity (R2, R5), and a worsening of skin return after extension (R1).

Comparing the data obtained for fertile women with those obtained for menopausal women, it can be seen that for the latter there is a decrease in skin compactness with an increase in maximum skin extensibility (R0menopause= 0.170 Vs R0fertile= 0.136), a decrease in resistance against returning to the resting condition (R2menopause= 0.573 Vs R2fertile= 0.821) and net elasticity (R5menopause= 0.348 Vs R5fertile= 0.794). The parameter regarding the ability of the skin to return to its initial state after being subjected to suction (R1 menopause= 0.794 Vs R1fertile= 0.348) also appears reduced.

Estrogens slow down the degradation of dermal collagen as they increase the transformation from its soluble form to the non-soluble cross-linked form. Deficient levels of estrogen lead to a reduction in type I collagen, the main responsible for skin thickness, and type III collagen, linked to skin elasticity. In menopausal women, indeed, there is a progressive reduction in skin thickness and an increase in skin atrophy.

The homogeneity of the dermis and its thickness have been assessed using an 22MHz DUB-system echograph (tpm-Taberna pro medicum, Luneburg, DE), having a resolution of 72µm. The instrument allows visualizing structures up to a depth of 8mm. This means that it is possible to evaluate the areas of diagnostic interest namely the epidermis, the dermis, and a portion of the subcutaneous adipose tissue. The dermis appears echogenic with echoes originating from the network of fibers consisting of collagen and elastic fibers; adipose accumulation and edema appear as hypoechoic areas. The thickness of the dermis is measured in µm.

The instrumental evaluation is performed at controlled temperature and humidity (T 22°C, R.H. = 50% ± 5%) whereat volunteers are acclimated for at least 15 minutes. The chosen acquisition area is the cheekbone.

40 subjects aged between 46 and 54 years were recruited as follows:
- FERTILE WOMEN:
   N.20 subjects who are in the three different phases of the menstrual cycle (random recruitment);
- WOMEN IN MENOPAUSE:
   N.20 subjects declare to be in menopause.

The following table (Tab.4) reports dermal thickness data.

| **Table 4: Average values of dermal thickness (µm) of all subjects** | | | |
|---|---|---|---|
| Group | Menstrual cycle phase | Age of subjects | Dermal thickness (µm) ± Std.Dev. |
| FERTILE WOMEN | Random | 38-53 | 2303.00 ± 250 |
| WOMEN IN MENOPAUSE | Absence | 51-58 | 1867.00 ± 128 |

The data obtained in the study confirm that the skin thickness in the recruited menopausal subjects decreases by about 19% when compared to the group of fertile age subjects.

Once the data from the preliminary study were obtained, an instrumental test was carried out to assess the efficacy of the procedure and the kit according to the invention, the design of which allowed a comparison with a conventional placebo cosmetic method to assess the actual impact of the present invention on a preliminary group of eight women for ten weeks of treatment based on the procedure according to the invention.

The results obtained, shown in Figs. 1-3, have demonstrated a tangible efficacy of the procedure and the kit, showing a substantial difference compared to the control group treated with a conventional placebo method/kit. Indeed, from the detailed comparison with the parametric fluctuations that naturally occur, it emerges that the cosmetic procedure according to the invention mitigates such variations, indicating a positive effect on the skin that manifests itself with the attenuation of fluctuations and/or the improvement of parameter variations. These results provide scientific evidence of the efficacy claimed by the procedure and the kit that corroborate the tangible benefit on female skin of fertile age subjects.

The data reported in the graphs of Figs. 1-3 are to be considered an exemplary summary of the treatment performance, through the functionalities related to the cosmetic procedure for enhancement and normalization and thus to the aforementioned kit.

In particular, the skin hydration data reflect the concepts of enhancing the beauty of the skin, while the sebum data and impurity count reflect the concepts of skin normalization.

Regarding skin hydration data of Fig. 1, expressed in arbitrary units (a.u.), show an increase in average hydration values after treatment based on the procedure/kit according to the invention for ten weeks, highlighting a significant improvement compared to the averages identified in the characterization studies, which are part of the scientific rationale of the procedure, and, more generally, a general improvement in skin condition. Indeed, if the parameter increases, this indicates an improvement.

Regarding sebum data of Fig. 2, expressed in µg/cm² and count of impurities, a reduction is observed after treatment based on the procedure/kit according to the invention for ten weeks, highlighting a significant improvement compared to the averages identified in the characterization studies which are part of the scientific rationale of the procedure, and, more generally, a minimization of natural differences and fluctuations. Indeed, a decrease in such parameters indicates an effective normalization of the skin condition, thus contributing to the overall improvement of the skin condition.

The results shown in the graph of Fig. 3 provide a clear indication of the efficacy of the cosmetic treatment based on the procedure and thus on the adoption of the kit according to the invention, highlighting the concepts of enhancement and normalization that are at the heart of the present invention.

The cosmetic procedure and the kit according to the invention thus offer a personalized approach to skin well-being, promoting a complete and targeted routine, whose modular nature makes it potentially versatile and adaptable to a diversified range of consumers. Indeed, the idea of a cosmetic procedure/kit inherently conceives flexibility of use, both in terms of cosmetic product forms and in terms of the site of application extendable to all skin parts or other skin appendages.

Therefore, the cosmetic procedure/kit for the optimization of aesthetic parameters of the skin and skin appendages according to the invention achieves significant advantages.

Indeed, cosmetic procedure for the optimization of aesthetic parameters of the skin and skin appendages and thus the said kit is capable of selectively counteracting the alterations of the skin and skin appendages caused, for example, by changes related to the menstrual cycle and/or by hormonal fluctuations in order to restore the ideal conditions of the skin and/or skin appendages.

Moreover, the cosmetic procedure/kit for the optimization of aesthetic parameters of the skin and skin appendages allows to relieve the consumer from his responsibilities, in particular by sparing him the task of understanding his own skin needs.

The invention is susceptible to variations within the scope of the inventive concept defined by the claims.

In this scope, all details are replaceable by equivalent elements and the materials, shapes, and dimensions can be any.

## Claims

1. Cosmetic procedure for the optimization of aesthetic parameters of the skin and skin appendages carried out over a period of 28 days,
- said period of 28 days comprising:
- a first week corresponding to a menstrual phase,
- a second week corresponding to a follicular phase,
- a third and fourth week corresponding to a luteal phase; and
- said procedure **being characterized by** including:
- during said first and fourth week, cleansing and treating said skin or said skin appendages with respectively a first mixture and a second mixture each including at least:
- polyphenolic structures contained in plant extracts,
- unsaponifiable substances of oils or fats,
- provitamins, vitamins and/or derivatives of group B; and
- during said second and third week, cleansing and treating said skin or said skin appendages with respectively a third mixture and a fourth mixture each including at least:
- biomimetic structures,
- natural or naturally derived polymers,
- unsaponifiable substances of oils or fats,
- provitamins, vitamins and/or derivatives of groups C and E,
- said second and fourth mixture further including Ectoin and Sodium Hyaluronate.

2. Procedure according to claim 1, wherein said biomimetic structures include one or more among glycolipids, phospholipids, amino acids, and peptides.

3. Procedure according to any of the preceding claims, wherein said polyphenolic structures are included in said first mixture in quantities ranging from 0.02% to 2% by weight of said first mixture, said unsaponifiable substances are included in said first mixture in quantities ranging from 0.02% to 5% by weight of said first mixture, and said provitamins, vitamins and/or derivatives of group B are included in said first mixture in quantities ranging from 0.05% to 10% by weight of said first mixture.

4. Procedure according to any of the preceding claims, wherein said polyphenolic structures are included in said second mixture in quantities ranging from 0.5% to 15% by weight of said second mixture, said unsaponifiable substances are included in said second mixture in quantities ranging from 0.2% to 10% by weight of said second mixture, said provitamins, vitamins and/or derivatives of group B are included in said second mixture in quantities ranging from 0.1% to 5% by weight of said second mixture, said Ectoin is included in said second mixture in quantities ranging from 0.2% to 5% by weight of said second mixture, and said Sodium Hyaluronate is included in said second mixture in quantities ranging from 0.02% to 10% by weight of said second mixture.

5. Procedure according to any of the preceding claims, wherein said biomimetic structures are included in said third mixture in quantities ranging from 0.5% to 30% by weight of said third mixture, said polymers are included in said third mixture in quantities ranging from 0.5% to 20% by weight of said third mixture, said unsaponifiable substances are included in said third mixture in quantities ranging from 0.5% to 10% by weight of said third mixture, and said provitamins, vitamins and/or derivatives of groups C and E are included in said third mixture in quantities ranging from 0.02% to 10% by weight of said third mixture.

6. Procedure according to any of the preceding claims, wherein said biomimetic structures are included in said fourth mixture in quantities ranging from 2% to 30% by weight of said fourth mixture, said polymers are included in said fourth mixture in quantities ranging from 0.02% to 15% by weight of said fourth mixture, said unsaponifiable substances are included in said fourth mixture in quantities ranging from 0.5% to 30% by weight of said fourth mixture, said provitamins, vitamins and/or derivatives of groups C and E are included in said fourth mixture in quantities ranging from 0.5% to 25% by weight of said fourth mixture, said Ectoin is included in said fourth mixture in quantities ranging from 0.01% to 5% by weight of said fourth mixture, and said Sodium Hyaluronate is included in said fourth mixture in quantities ranging from 0.1% to 5% by weight of said fourth mixture.

7. Procedure according to any of the preceding claims, wherein said first mixture includes water of flowers/leaves/stems of at least one among Angelica and Melissa.

8. Procedure according to any of the preceding claims, wherein said second mixture includes Melissa officinalis flower/leaf/stem water, Vitis vinifera seed oil, Helianthus annuus seed oil, punica granatum extract, oleanolic acid, jojoba esters, Angelica archangelica extract, Melissa officinalis extract, Euphorbia cerifera wax, Plantago lanceolata extract, Mahonia aquifolium extract, Enantia chlorantha extract, xanthan gum, yogurt extract, sodium salicylate.

9. Procedure according to any of the preceding claims, wherein said third mixture includes Olea europaea oil, Vitis vinifera seed oil, glycolipids, Helianthus annuus seed oil, Curcuma longa extract, sodium PCA, tocopherol, xanthan gum, potassium olivoyl PCA.

10. Procedure according to any of the preceding claims, wherein said fourth mixture includes flowers/leaves/stems water of Melissa officinalis, Helianthus annuus seed oil, Pistacia vera seed oil, Ribes nigrum extract, jojoba esters, xanthan gum, Euphorbia cerifera wax.

11. Cosmetic kit for the optimization of aesthetic parameters of the skin and skin appendages **characterized by** including:
- a first mixture and a second mixture each including at least:
- polyphenolic structures contained in plant extracts,
- unsaponifiable substances of oils or fats,
- provitamins, vitamins and/or derivatives of group B; and
- a third mixture and a fourth mixture each including at least:
- biomimetic structures,
- natural or naturally derived polymers,
- unsaponifiable substances of oils or fats,
- provitamins, vitamins and/or derivatives of groups C and E, and **the fact that**
- said second and fourth mixture further include Ectoin and Sodium Hyaluronate.

12. Kit according to the previous claim, wherein
- said first mixture includes:
- said polyphenolic structures in quantities ranging from 0.02% to 2% by weight of said first mixture,
- said unsaponifiable substances in quantities ranging from 0.02% to 5% by weight of said first mixture, and
- said provitamins, vitamins and/or derivatives of group B in quantities ranging from 0.05% to 10% by weight of said first mixture;
- said second mixture includes:
- said polyphenolic structures in quantities ranging from 0.5% to 15% by weight of said second mixture,
- said unsaponifiable substances in quantities ranging from 0.2% to 10% by weight of said second mixture,
- said provitamins, vitamins and/or derivatives of group B in quantities ranging from 0.1% to 5% by weight of said second mixture,
- said Ectoin in quantities ranging from 0.2% to 5% by weight of said second mixture, and
- said Sodium Hyaluronate in quantities ranging from 0.02% to 10% by weight of said second mixture;
- said third mixture includes:
- said biomimetic structures in quantities ranging from 0.5% to 30% by weight of said third mixture,
- said polymers in quantities ranging from 0.5% to 20% by weight of said third mixture,
- said unsaponifiable substances in quantities ranging from 0.5% to 10% by weight of said third mixture, and
- said provitamins, vitamins and/or derivatives of groups C and E in quantities ranging from 0.02% to 10% by weight of said third mixture
- said fourth mixture includes:
- said biomimetic structures in quantities ranging from 2% to 30% by weight of said fourth mixture,
- said polymers in quantities ranging from 0.02% to 15% by weight of said fourth mixture,
- said unsaponifiable substances in quantities ranging from 0.5% to 30% by weight of said fourth mixture,
- said provitamins, vitamins and/or derivatives of groups C and E in quantities ranging from 0.5% to 25% by weight of said fourth mixture,
- said Ectoin is included in said fourth mixture in quantities ranging from 0.01% to 5% by weight of said fourth mixture, and
- said Sodium Hyaluronate is included in said fourth mixture in quantities ranging from 0.1% to 5% by weight of said fourth mixture.

13. Kit according to the previous claim, wherein said first mixture includes flowers/leaves/stems water of at least one among Angelica and Melissa; said second mixture includes Melissa officinalis flower/leaf/stem water, Vitis vinifera seed oil, Helianthus annuus seed oil, punica granatum extract, oleanolic acid, jojoba esters, Angelica archangelica extract, Melissa officinalis extract, Euphorbia cerifera wax, Plantago lanceolata extract, Mahonia aquifolium extract, Enantia chlorantha extract, xanthan gum, yogurt extract, sodium salicylate; said third mixture includes Olea europaea oil, Vitis vinifera seed oil, glycolipids, Helianthus annuus seed oil, Curcuma longa extract, sodium PCA, tocopherol, xanthan gum, potassium olivoyl PCA; and said fourth mixture includes flowers/leaves/stems water of Melissa officinalis, Helianthus annuus seed oil, Pistacia vera seed oil, Ribes nigrum extract, jojoba esters, xanthan gum, Euphorbia cerifera wax.
